# EUROPEAN PATENT APPLICATION

(11) **EP 4 682 537 A1**
(43) Date of publication of application: **21.01.2026**
(21) Application number: 24774731.4
(22) Date of filing: 08.03.2024
(51) Int. Cl.: G01N 33/50

(54) **LIVING BODY INSPECTION METHOD, INSPECTION SYSTEM, AND PATCH**

(30) Priority: 20.03.2023 JP 2023044496
(71) Applicant: Pittan Inc., Kobe-shi, Hyogo 657-0831 (JP)
(72) Inventor: TSUJIMOTO, Kazuya, Kobe-shi, Hyogo 657-0831 (JP)
(74) Representative: Isarpatent
(86) International application number: PCT/JP2024/009147
(87) International publication number: WO 2024/195592

(57) **Abstract**

The present invention makes it possible to acquire biological information relating to an examination subject more easily or more accurately and to provide the biological information to a user. This living organism examination method for providing biological information relating to an examination subject (1), which is a living organism , by analyzing biological components in the sweat of the examination subject includes: a collection step (S101) for collecting sweat from an examination site of the examination subject (1); a measurement step (S102) for measuring a biological component profile of the sweat collected in the collection step; a biological information acquisition step (S103) for acquiring biological information relating to the examination subject (1) on the basis of the biological component profile measured in the measurement step; and an information provision step (S104) for providing the biological information relating to the examination subject , acquired in the biological information acquisition step, in a predetermined format.

## Description

### [FIELD]

The present invention relates to an examination method, a patch used in said examination method, and an examination system, for acquiring biological information of a subject by analysing biological components (amino acids, proteins, bases, lipids, extracellular vesicles, etc.) contained in sweat of the subject.

### [BACKGROUND]

In recent years, due to increased awareness of anti-aging and healthcare, more people are using dietary supplements and health-related cosmetics. To effectively utilise these supplements, it is important to continuously monitor biological information derived from daily lifestyle habits. However, information obtained from the outside of the body, such as weight, blood pressure, electromyogram, electrocardiogram, and pulse, are insufficient. It is essential to understand the internal state of the body conditions, including deficient nutrients, the state of biological metabolites, and changes in the balance of multiple biological components.

For early diagnosis of various pathological conditions, biomarkers are sometimes explored from biomolecules in the blood. However, blood collection is invasive and burdensome for subjects, and it is not easy to perform repeatedly over time. Urine is a non-invasive sample but cannot always be collected on demand. As a solution, sweat, which can be collected anytime, is gaining attention for component analysis using wearable devices. Nevertheless, current wearable sensors measure only a limited range of components, such as glucose and sodium ions, and much remains unknown about the other biological components in sweat.

Thus, in order to realise personalised healthcare based on internal "wet" component information, research has been conducted on methods for analysing the components in body fluids (such as blood, urine, tears, saliva, and sweat) secreted or excreted by the body, as well as on wearable devices. However, conventional methods are limited in the components they can measure, and often involve high physical or psychological invasiveness, making it difficult to acquire necessary biological information in a minimally invasive, simple, and rapid manner.

### [Prior Art]

[Patent Literature 1] International Publication No. 2021/246074
[Patent Literature 2] Japanese Patent Application Publication No. 2010-48791
[Patent Literature 3] Japanese Patent Application Publication No. 2021-120627
[Patent Document 4] Japanese Patent Application Publication No. 2022-119774

### [SUMMARY]

The present invention was conceived in light of the above circumstances, and its purpose is to enable the acquisition of biological information of the subject in an easier and more accurate manner and the provision of the biological information to users.

In order to achieve the aforementioned objectives, the present disclosure adopts the following configuration. That is, a method of examining a living organism and providing biological information on an examination subject by analyzing biological components in sweat of the examination subject which is the living organism, the method comprising:
a collection step of collecting the sweat at an examination site in the examination subject;
a measurement step of measuring biological components profile of the sweat collected in the collection step;
a biological information acquisition step of acquiring, based on the biological components profile measured in the measurement step, the biological information on the examination subject; and
an information provision step of providing the biological information on the examination subject, acquired in the biological information acquisition step, in a predetermined format.

According to this method, since sweat-a bodily fluid that can be collected without constraints on location or time, unlike urine or blood-is used to measure the biological component profile and acquire biological information about the subject, the biological information can be provided to users in an easily understandable format. As a result, the users can more easily obtain biological information about the subject. The method of collecting sweat in the aforementioned collection step may include using a patch as described later but is not limited thereto.

In addition, in the present disclosure, the biological information on the examination subject may include information on a state of a skin or a muscle of the examination subject at the examination site, the information being acquired based on the biological components profile.

This enables easy acquisition of information about the skin at the examination site where the sweat was collected, based on the biological component profile of the collected sweat. Notably, since sweat is a bodily fluid that passes through the skin before being excreted externally, it inherently contains information about the skin. Therefore, this disclosure allows for more accurate acquisition of skin-related information. Additionally, by collecting sweat before, during, and after physical activity involving specific muscles, it becomes possible to obtain a biological component profile that includes information about those muscles. Consequently, biological information related to desired muscles can be acquired with higher accuracy.

In addition, in the present disclosure, the biological information on the examination subject may include information on a state of any one of disease, sleep, and inflammation of the examination subject, the information being acquired based on the biological components profile.

This enables easy acquisition of information regarding any of the aforementioned conditions-diseases, sleep, or inflammation-based on the biological component profile in the collected sweat.

In addition, in the present disclosure, the biological information of the test subject may include biological information of the test subject obtained from the amounts of the biological components that make up the biological component profile. In this case, the relationship between the quantity of each biological component in the biological component profile and the subject's biological information enables easy acquisition of the subject's biological information. The relationship may be pre-established and stored in a database.

In addition, in the present disclosure, the biological information on the examination subject may include biological information on the examination subject, which is acquired by analyzing, based on a structural equation modelling method, the biological components profile.

By analysing the measured biological component profile through SEM, it becomes possible to acquire the subject's biological information that lies behind the biological component profile. This allows the system to provide users with deeper insights into the subject's mental and physical health based on statistical analysis of the biological component profile.

In addition, in the present disclosure, the biological information on the examination subject may include biological information on the examination subject, which is acquired by analyzing the biological components profile by machine learning. This also enables the acquisition of the subject's biological information that lies behind the biological component profile and allows the system to provide users with deeper insights into the subject's mental and physical health. Machine learning, in this context, encompasses concepts such as artificial intelligence (AI) and deep learning. The computing devices for machine learning may include personal computers, supercomputers, or quantum computers, without limitation.

In addition, the biological information on the examination subject may include an amount of an individual biological component constituting the biological components profile, and the information provision step provides information on at least one of a type of an insufficient or excessive biological component in the examination subject and a method of resolving the insufficiency or excess.

Based on analysis of the measured biological component profile, it becomes possible to provide the user with information about which biological components are lacking or excessive in the subject, and methods to correct such imbalances. The methods may include the introduction of specific foods or supplements that effectively supply the necessary biological components.

In addition, in the present disclosure may further include a memory step of storing the biological information on the examination subject in association with the examination subject; and a set information provision step of providing, as a set, the biological information for a set of a plurality of the examination subjects.

According to this disclosure, information such as amino acid profiles in subject's sweat and biological information of many subjects can be collected and stored in association with the respective subjects. The resulting dataset, once aggregated, becomes a valuable resource from which various new insights can be statistically derived. This disclosure allows the construction of such high-value data clusters and enables their provision to users and third parties.

In addition, in the present disclosure, in the collection step, the sweat may be collected from the examination subject by attaching a patch to the examination site, wherein the patch includes a moisture-absorbing portion capable of absorbing liquid, and an adhesive sheet having a larger area than an area of the moisture-absorbing portion, the adhesive sheet having an adhesive surface on at least one surface thereof, the adhesive surface covering the moisture-absorbing portion such that the moisture-absorbing portion is held to be attachable to the examination site.

Through the inventors' intensive research, it was found that when collecting sweat samples from subjects, a patch with a moisture-absorbing portion capable of absorbing liquids and a structure covering the moisture-absorbing portion with an adhesive surface that has a larger area than the moisture-absorbing portion can be applied to the examination site to effectively collect sweat from the subject. With this method, it is possible for examiners without specialised knowledge or the subjects themselves to collect sweat more easily and reliably.

In addition, in the present disclosure, the moisture-absorbing portion may be moistened with water before the patch is attached to the examination site. In addition to the above findings, the inventors have clarified that sweat can be collected more reliably by pre-moistening the moisture-absorbing portion of the patch with water before attaching the patch to the examination site. This method makes it possible to more reliably collect sweat from the subject in the collection step.

In addition, in the collection step, an attachment location of the patch may be any one of a cheek, a temple, a forehead, a back side of a mandibular angle, and a rear side of a neck of the examination subject, or a combination thereof. Here, in the present disclosure, it has been found that when sweat is collected from the subject, the components of the sweat collected from the cheek, temple, and forehead are similar. Therefore, even if there is a site where it is difficult to collect sweat due to the inadequate amount of sweat produced by the subject or the condition of the site , it is possible to perform highly accurate measurement if sweat can be collected from any of the cheek, temple, and forehead. **In** addition, the back side of the mandibular angle (so-called jaw) and the rear side of the neck are sites where foreign matter such as cosmetics, beauty essences, and medicines are unlikely to be attached, or even if they are attached, the subject will have relatively little resistance to removing them, and by using these sites as sweat collection sites, it is possible to more easily collect sweat that does not contain foreign matter.

In addition, in the present disclosure, the patch after the sweat is collected in the collection step may be moved in a state in which the patch is attached to an inside of a bottom surface of a flat bottomed cylindrical case with one side closed, and the measurement step is performed. In this way, the collected sweat is retained between the case bottom and the adhesive sheet, minimising contamination. This setup also prevents the patches from coming into contact with one another when multiple samples are transported, thereby avoiding cross-contamination. Here, "cylindrical" includes not only circular cylinders but also other cross-sectional shapes such as polygons, ellipses, ovals, or even open shapes.

In addition, the biological components may include at least one of amino acids, proteins, bases, lipids, and extracellular vesicles.

In addition, in the present disclosure, a patch for collecting sweat may be a patch adapted to collect, in the collection step of the method of examining a living organism, the sweat at the examination site in the examination subject, the patch comprises a moisture-absorbing portion capable of absorbing liquid, and an adhesive sheet having a larger area than an area of the moisture-absorbing portion, the adhesive sheet having an adhesive surface on at least one surface thereof, the adhesive surface covering the moisture-absorbing portion such that the moisture-absorbing portion is held to be attachable to the examination site.

In this disclosure, sweat can be collected by attaching the patch to the desired site of subject's skin and maintaining the patch in place for a predetermined period. With the patch of the configuration described above, sweat can be collected more easily from the subject's skin. Once sweat collection is complete, by peeling and retrieving the patch from the skin, sweat sample can be stored or transported more easily. Before analysis, by extracting the biological components (e.g., amino acids, proteins, bases, lipids, extracellular vesicles) from moisture-absorbing portion of the patch into a solution, a sample for profile measurement can be prepared. The moisture-absorbing portion may be made of filter paper, water-absorbing polymer, or similar materials. Although sheet form is typical, other shapes are also acceptable.

In the present disclosure, the patch may further include a separation mechanism configured to facilitate, when a part of the moisture-absorbing portion is cut off to prepare a sample in the measurement step, separation of the part of the moisture-absorbing portion.

This enables a part of the moisture-absorbing portion containing the sweat to be separated from the peeled off patch and used in an optimal form for preparing a sample for profile measurement. The separation mechanism may be, for example, perforations formed in the moisture-absorbing portion and the adhesive sheet and in easily handled shapes such as circles or polygons. Alternatively, the moisture-absorbing portion may have a layered structure made of two stacked sheets of filter paper, allowing one sheet to be peeled away and separated from the patch for sample preparation.

In addition, in the present disclosure, the patch may further include a holding portion capable of holding the adhesive sheet when the adhesive sheet is peeled off from the examination site. This enables the user to peel off the patch applied to the examination site while holding the holding portion. Consequently, it becomes easier to collect sweat using the patch and prevents the user's fingers from touching the moisture-absorbing portion during removing and retrieving, which helps avoid contamination from finger sweat. As a result, sweat from only the examination site can be collected with greater accuracy.

In addition, in the present disclosure, the patch may further include a base sheet adapted to protect the moisture-absorbing portion by allowing the adhesive surface of the adhesive sheet to be attached to the base sheet before and after use; and a bag-shaped moisture supply container adapted to contain collection liquid therein, the moisture supply container being disposed between the moisture-absorbing portion and the adhesive sheet or between the moisture-absorbing portion or the adhesive sheet and the base sheet, wherein, when the adhesive sheet and the moisture-absorbing portion are peeled off from the base sheet, the moisture supply container is broken to enable moisture supply to the moisture-absorbing portion.

According to this, just before attaching the patch to the subject to collect sweat, the moisture-absorbing portion is supplied with the collection liquid to moisten the moisture-absorbing portion and make the moisture-absorbing portion easier to collect sweat. As a result, even if the amount of sweat to be collected is small, evaporation can be suppressed and sweat can be collected more reliably. The collection liquid here may be water, an organic solvent, a mixed liquid adjusted with other components, or a combination of these.

In addition, in the present disclosure may also provide a storage container adapted to store therein the patch, the storage container includes an sealing portion adapted to, when the storage container is deformed, suppress drying of the moisture-absorbing portion by allowing an inner surface of the sealing portion to contact the moisture-absorbing portion or the adhesive sheet.

This configuration allows the retrieved patch to be stored in a storage container and transported to the place where the biological component profile will be measured, preventing drying of the moisture-absorbing portion and contamination by foreign matters. Furthermore, the storage container is capable of maintaining contact between the inner wall and the moisture-absorbing portion or the adhesive sheet when deformed, ensuring even more reliable protection against drying or contamination. As a result, the accuracy of biological component profile measurements can be improved. Particularly in cases where the biological component is an extracellular vesicle, drying after collection makes profile measurement impossible, so preventing drying of the moisture-absorbing portion using the container with the above structure enables successful profile measurement.

In addition, present disclosure may be a storage container includes a separation assist mechanism configured to, when the storage container is deformed, assist separation of a part of the moisture-absorbing portion by the separation mechanism.

This allows a part of the moisture-absorbing portion to be separated while the patch remains in the storage container, making it easier to prepare the sample for measurement. This prevents contact between fingers and the moisture-absorbing portion during separating the moisture-absorbing portion from the patch, thereby avoiding contamination with finger sweat and ensuring that sweat from the desired examination site is reliably collected.

In addition, present disclosure may be an examination system for a living organism, configured to provide biological information on an examination subject by analyzing biological components in sweat of the examination subject which is the living organism, the examination system, which includes a collection tool configured to collect the sweat at an examination site in the examination subject, a measurement unit configured to measure a biological components profile of the sweat collected by the collection tool, a biological information acquisition unit configured to acquire, based on the biological components profile measured by the measurement unit, the biological information on the examination subject, and an information provision unit configured to provide the biological information on the examination subject, acquired by the biological information acquisition unit, in a predetermined format.

In addition, in the examination system for a living organism, the biological information on the examination subject may include information on a state of a skin or a muscle of the examination subject, the information being acquired based on the biological components profile. In addition, the biological information on the examination subject may include information on any one of disease, sleep, and inflammation of the examination subject, the information being acquired based on the biological components profile.

In addition, in the examination system for a living organism, the biological information on the examination subject may include biological information on the examination subject, which is acquired from an amount of the biological components constituting the biological components profile. In addition, the biological information on the examination subject may include biological information on the examination subject, which is acquired by analyzing, based on a structural equation modelling method, the biological components profile. In addition, the biological information on the examination subject may include biological information on the examination subject, which is acquired by analyzing the biological components profile by machine learning.

In addition, in the examination system for a living organism, the biological information on the examination subject may include an amount of an individual biological component constituting the biological components profile, and the information provision unit further provides information on at least one of a type of an insufficient or excessive biological component in the examination subject and a method of resolving the insufficiency or excess.

In addition, the examination system for a living organism may further include, a storage unit configured to store therein the biological information on the examination subject in association with the examination subject, and a set information provision unit configured to provide, as a set, the biological information for a set of the plurality of examination subjects.

In addition, in the examination system for a living organism, the collection tool may have a moisture-absorbing portion capable of absorbing liquid, and the sweat is collected by attaching the collection tool to the examination subject such that the moisture-absorbing portion that has absorbed collection liquid in advance comes into contact with the examination site.

In addition, in the examination system for a living organism, the biological components may include at least one of amino acids, proteins, bases, lipids, and extracellular vesicles.

Each of the aforementioned configurations and processes may be combined freely, provided they do not introduce technical inconsistencies, to constitute the invention.

According to the present invention, it is possible to obtain the biological information of a subject more easily or more accurately and provide the biological information to a user.

### [BRIEF DESCRIPTION OF THE DRAWINGS]

[Figure 1] This figure shows an overview of the examination method for living organisms related to the embodiment of this disclosure.
[Figure 2] This figure explains the importance of amino acids in the health and beauty of living organisms.
[Figure 3] This figure is a flowchart of the biological examination method for living organisms related to the embodiment of this disclosure.
[Figure 4] This figure is a conceptual diagram of the measurement principle of the liquid chromatograph device related to the embodiment of this disclosure.
[Figure 5] An example of an amino acid profile obtained by the liquid chromatograph device related to the embodiment of the present invention.
[Figure 6] An example of a path diagram of a structural equation model for acquiring biological information related to the embodiment of the present invention.
[Figure 7A and Figure 7B] Examples of information provided for viewing on a smartphone app related to the embodiment of the present invention.
[Figure 8] Another example of information provided for viewing on a smartphone app related to the embodiment of the present invention.
[Figure 9] This is a functional block diagram of an embodiment of the present invention when viewed as an examination system for living organisms.
[Figure 10] This is an example of an actual operational form of an examination method or examination system according to an embodiment of the present invention.
[Figure 11A and Figure 11B] These are diagrams showing the first specific structure of a sweat-absorbing patch according to an embodiment of the present invention.
[Figure 12A and Figure 12B] These are diagrams showing the second specific structure of a sweat-absorbing patch according to an embodiment of the present invention.
[Figure 13A and Figure 13B] These are diagrams showing the third specific structure of a sweat-absorbing patch according to an embodiment of the present invention.
[Figure 14A and Figure 14B] These are diagrams showing the fourth specific structure of a sweat-absorbing patch according to an embodiment of the present invention.
[Figure 15A and Figure 15B] These are diagrams showing the fifth specific structure and usage of a sweat-absorbing patch according to an embodiment of the present invention.
[Figure 16A and Figure 16B] Another diagrams showing the fifth specific structure and usage of the sweat-absorbing patch according to an embodiment of the present invention.
[Figure 17] A graph showing the detection amounts of each amino acid when the moisture-absorbing sheet according to an embodiment of the present invention is moistened with water before sweat collection and when it is not moistened.
[Figure 18A and Figure 18B] These figures show the amino acid profiles obtained when the moisture-absorbing sheet according to an embodiment of the present invention is moistened with water before collecting sweat and when it is not moistened.

### [DETAILED DESCRIPTION]

The following is a description about embodiments of the invention based on the drawings. However, the elements described in each of the following embodiments are not intended to limit the scope of this invention to the elements alone, unless otherwise noted.

### <Embodiment>

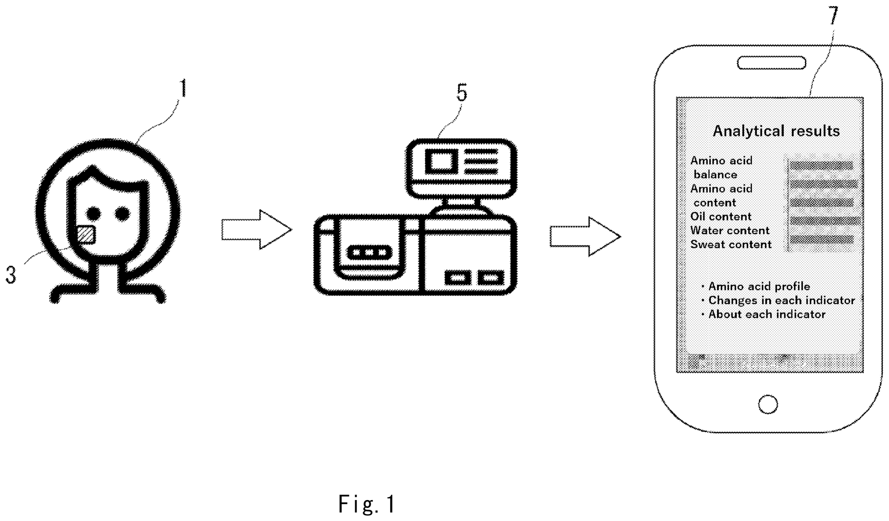

Figure 1 shows an overview of the method for examining living organisms in this embodiment. In this embodiment, examining living organisms means getting the biological information which includes the information on nutritional status, metabolism, potential mental and physical condition etc. In this embodiment, we will talk about when the living organism as subject 1 is a person. However, the subject 1 is not limited to humans and may also be other animals.

In the examination method of this embodiment, sweat from the subject 1 is first collected as a sample. The collection method is shown in Figure 1, where sweat is collected from the skin of the subject 1 using the patch 3 with moisture-absorbing. Here, "moisture-absorbing" refers to either or both of the properties of water absorption and oil absorption. The collected sweat is then performed to separation analysis in the analysis device 5. In this embodiment, the analysis device 5 is a liquid chromatograph, but the method of separation analysis of sweat is not limited to liquid chromatography. The results of the separation analysis and associated information are then provided to the user in a format such as that shown on the display screen 7. In this embodiment, we're talking about using the patch 3 as a device to collect sweat but sweat can be collected in other ways too. For example, it could be collected directly using a collection liquid. Here, the collection liquid means water, an organic solvent, or a mixture of other components. This also applies to the following descriptions in this specification.

In the examination method in this embodiment, a sample can be collected at any time and any place by simply attaching the patch 3 to the examination site for several minutes. In addition, since the biological component in sweat does not easily fluctuate in the short term, stable results can be obtained. In addition, since the biological component in sweat is collected by the patch moistened with the collection liquid, both hydrophilic and lipophilic components can be handled depending on the component of the collection liquid. In Figure 1, the results of the separation analysis are provided to the user by displaying them on a terminal such as a smartphone, but the medium for providing information is not limited to this. The information may also be provided by telephone or paper media. The user of this examination method does not necessarily correspond to the subject 1. The user may be a third party different from the subject 1. Although this embodiment describes analysing amino acids in sweat as biological components, this disclosure also includes collecting and analysing proteins, bases, lipids, extracellular vesicles, etc. other than amino acids as biological components. This disclosure also includes collecting and analysing proteins, bases, lipids, extracellular vesicles, etc., as biological components.

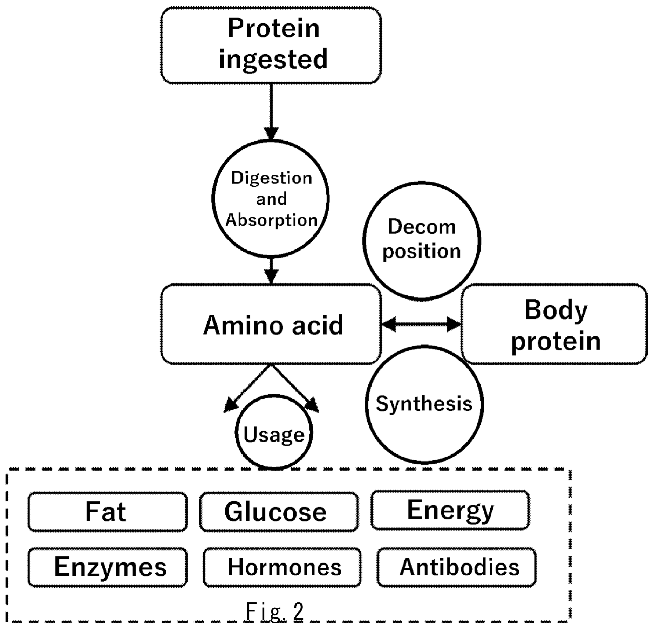

Next, using Figure 2, we will show the importance of amino acids in the health and beauty of living organisms. Proteins ingested by living organisms through food are digested and absorbed by the body and converted into amino acids. Within the body, these amino acids are used to synthesise substances necessary for life activities, such as fats, glucose, enzymes, hormones, and antibodies, as well as to generate energy for life activities. In addition, the body proteins that make up living organisms are synthesised based on amino acids. Amino acids and body proteins within the body maintain equilibrium state through synthesis and decomposition. For example, when the amount of amino acids in the body decreases, muscles may be broken down to generate amino acids.

It is known that amino acids in the human body are deeply related to skin condition. For instance, approximately half of the natural moisturising factors (NMF) that hydrate the stratum corneum consist of amino acids. Additionally, keratinocytes that support the NMF within corneal cells are also composed of amino acids. Collagen, which supports the firmness and elasticity of skin, is also made from amino acids. In this way, amino acids are closely linked to both the health and beauty conditions of the human body. Therefore, by analysing the state of amino acids inside the body, it becomes possible to obtain information indicative of the subject's health and beauty conditions.

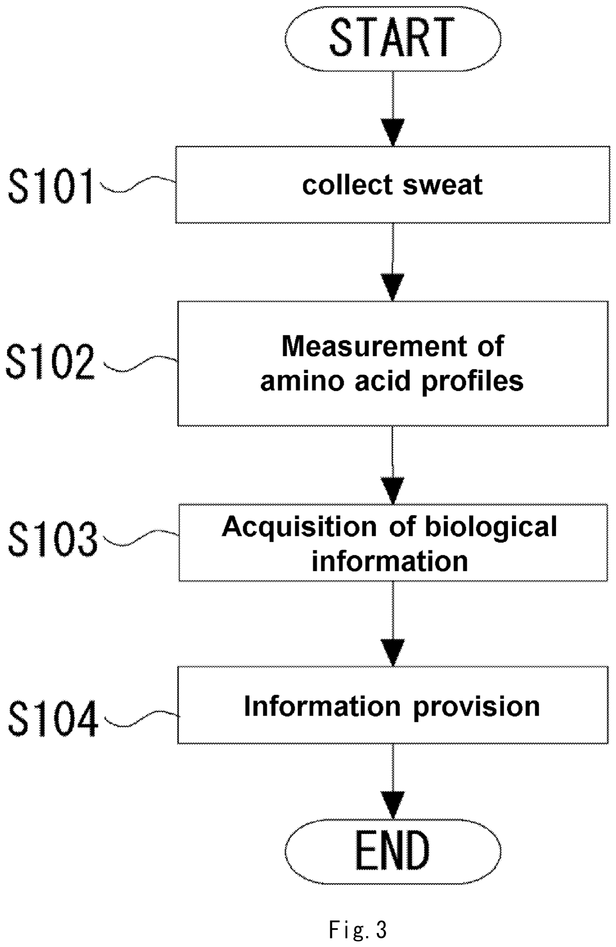

Next, we will explain in more detail the method for examining living organisms in this embodiment. Figure 3 shows a flowchart of the method for examining living organisms in this embodiment. When this flow is executed, first, in step S101, which corresponds to the collection step, sweat sample is collected from the subject 1. Specifically, for example, a patch 3 containing an absorbent sheet such as filter paper, non-woven fabric, or gauze is attached to a predetermined examination site (face, hands, etc.) on the subject 1, and the patch is maintained in place for a predetermined period of time. This allows sweat generated by sweating from the subject 1 to be absorbed and collected by the patch 3. Here, the longer the predetermined time, the higher the precision of amino acid separation in the subsequent amino acid profile measurement; however, the specified time may be as short as a few minutes.

The amount of sweat collected, for example, a few µl, is sufficient for a highly accurate examination. When the patch 3 is attached to the face, it can be attached to the cheek, temple, forehead, the back side of the mandibular angle (the so-called jaw), the rear side of the neck, or any combination thereof. The components of sweat collected from the cheeks, temples, and forehead are similar. In addition, the back side of the mandibular angle (jaw) and the rear side of the neck are sites where there is little possibility of foreign matters such as cosmetics, serums, or drugs adhering, or where the subject has relatively little resistance to remove the foreign matters if they do adhere, and sites where sweat is easily collected. Therefore, it is possible to collect sweat sample more reliably by using the above-mentioned sites as the place where the patch 3 is attached. The specific structure of the patch 3 is described below. After step S101 is completed, the process proceeds to step S102.

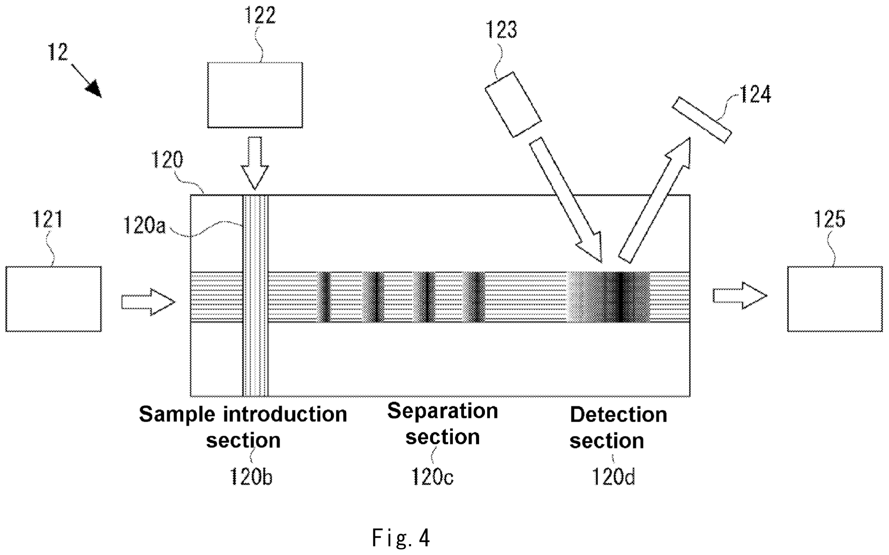

In step S102, which corresponds to the measurement step, the amino acid profile is measured by the liquid chromatograph device 12 as the analysis device 5 using the patch 3 containing sweat sample. Figure 4 shows a conceptual diagram of the measurement principle in the liquid chromatograph device 12. In Figure 4, the column (stationary phase) 120 has a structure in which a carrier such as diatomaceous earth is packed in a container. By passing the sample generated by fluorescent modification to the components in the collected sweat through the sample path 120a in the column 120, the amino acids in the sweat can be separated by type. In liquid chromatography, a mobile phase 121, a liquid for separation of amino acids in sweat, flows through the column 120. This mobile phase 121 flows at a constant rate through the column 120 (stationary phase) from the sample introduction section 120b to the detection section 120d by means of a pump, which is not shown in the figure.

Then, the sample 122 is injected into the sample introduction section 120b and is put into the flow of the mobile phase 121 in the column 120. Then, the amino acids in the sweat move through the separation section 120c in the column 120 by the flow of the mobile phase 121, and since the moving speed differs depending on the type of amino acid, the amino acids are separated in the separation section 120c. Each separated amino acid is detected by detecting the fluorescence generated by each amino acid by the excitation light irradiated from the light source 123 with the light receiving element 124. Then, the type of each amino acid is identified by the time at which each amino acid is detected in the detection section 120d, and the amount of each amino acid is detected from the fluorescence intensity. As a result, qualitative and quantitative analysis of the type of amino acid is possible. In addition to the fluorescence detection of the amino acids in the detection section 120d, or instead of the fluorescence detection of the amino acids in the detection section 120d, an analysis such as mass spectrometry may be performed in the external detector 125.

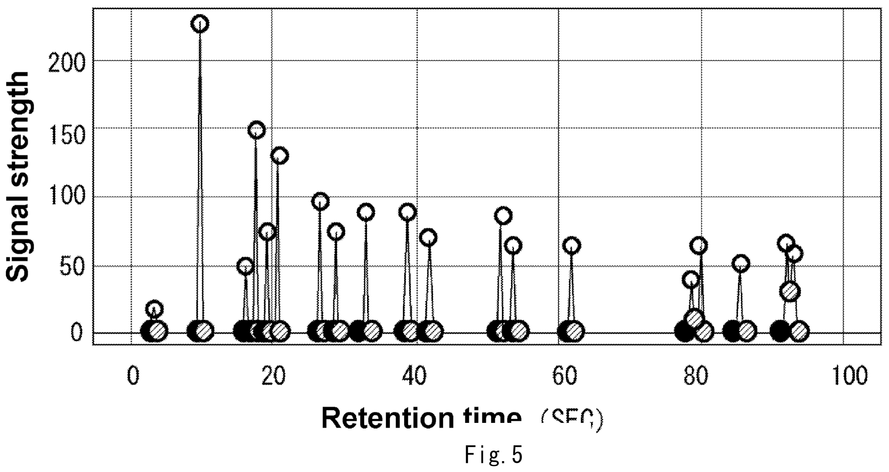

Figure 5 shows an example of an amino acid profile obtained using the liquid chromatograph device 12. The horizontal axis in Figure 5 represents the retention time for each type of amino acid in the column, which reflects the migration speed of each amino acid.

When the processing of step S102 is completed, the process proceeds to step S103. In step S103, which corresponds to the biological information acquisition step, biological information is acquired. More specifically, by analysing the amino acid profile measured in step S102, the types of amino acids that are deficient or excessive in the subject 1 and the degree of deficiency or excess are detected.

In analysing the amino acid profile, a reference sample containing the amino acid to be detected is used in advance in the liquid chromatograph 12 to measure the reference amino acid profile. In analysing the amino acid profile measured in step S102, the peak that appears at the closest time to the peak corresponding to each amino acid in the reference amino acid profile is determined to be the peak of that amino acid. The determination of the peak corresponding to each amino acid may be performed automatically by software using data of each peak in the reference amino acid profile that is stored in advance in the memory of the analysis device.

In this case, it is desirable to use amino acid profile data that is as recent as possible (e.g., with a time difference of no more than two days from the actual analysis). This reduces errors caused by changes in the liquid chromatograph device 12 over time and environmental changes, thereby improving the accuracy of the analysis. Furthermore, through amino acid profile analysis, it is possible to obtain information not only about the condition of the skin and muscles of the subject 1, but also about the presence or absence of allergies or mental disorders. Additionally, information related to diseases, sleep, or inflammation that are hidden behind the amino acid profile (i.e., the causes of the obtained amino acid profile) can also be obtained. Furthermore, using structural equation modelling techniques, machine learning, deep learning, or Al, it is possible to perform more detailed analysis of the obtained amino acid profile.

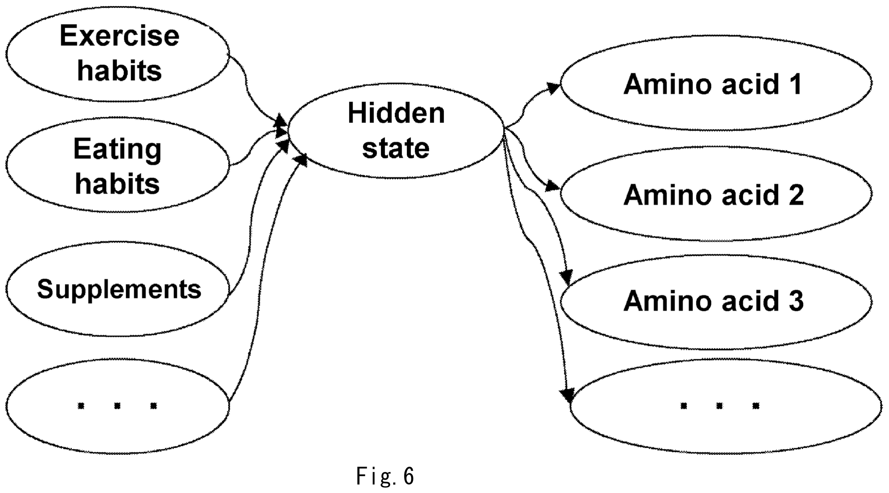

Figure 6 is an example of a path diagram of a structural equation model for obtaining biological information including the physical and mental state of the subject 1, such as diseases, which are hidden behind the amino acid profile of the subject 1. In this embodiment, the influence relationship between exercise habits, eating habits, type of supplements being taken, etc. as observed variables, hidden states (i.e., diseases, etc.) as latent variables, and the amount of each amino acid as observed variables are identified. In step S103, the relationship between factors such as exercise habits, eating habits, supplements, etc., the amount of each amino acid, and diseases as hidden states is identified by this structural equation modelling method. Furthermore, the relationship is learned and used to obtain the hidden state of mind and body from the factors such as exercise habits, eating habits, supplements, etc., and the amino acid profile.
Upon completion of step S103, the process proceeds to step S104.

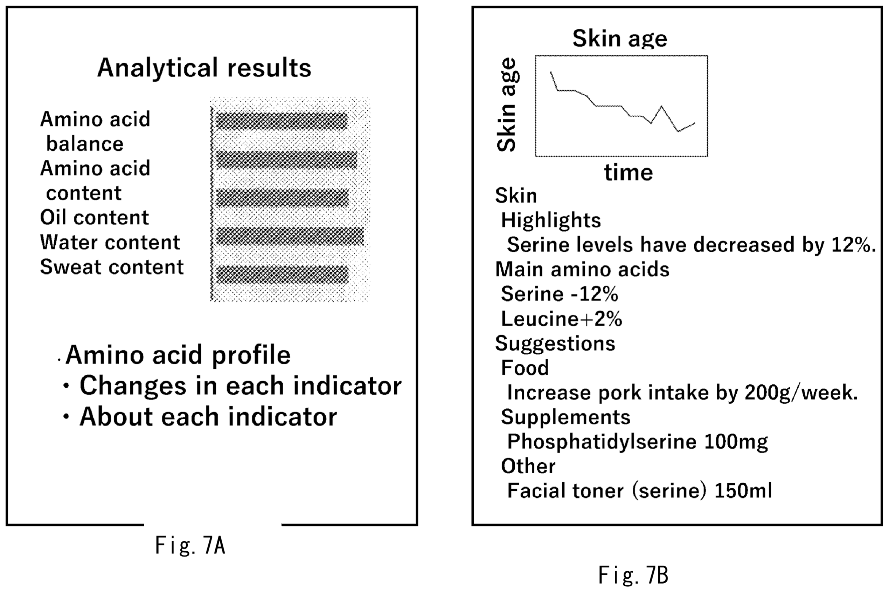

In step S104, which corresponds to the information provision step, the information obtained in S103 is presented to the user. Specifically, the information obtained in S103 is uploaded to a server and made accessible through an application or web app previously provided to the user. Figure 7A is an example of a display screen for the analysis results of the amino acid profile measured in step S102. Figure 7B is an example of a display screen that includes information on changes in skin age and suggestions on how to supplement the missing amino acids. In addition, information about diseases that are hidden behind the scenes, obtained in step S103, may be displayed. Once the process in step S104 is completed, this flow ends once and for all. Here, the formats of providing information as illustrated in Figure 7A and Figure 7B correspond to the prescribed format in this disclosure. The information illustrated in Figure 7A and Figure 7B are all obtained from sweat that has passed through the skin of the subject and include information on the condition of the skin.

In Figure 7A and Figure 7B, the oil content, water content, skin age, etc. are listed as indicators related to the condition of the skin of the subject 1, but original indicators such as "Moisturizing power", "Anti-sagging power", "Barrier power", "Redness", "Skin firmness retention power", "Softness", "Facial expression muscles (FIM)", etc. may be used as indicators related to the condition of the skin. The "Moisturizing power" may be related to the ability to retain moisture and means the skin's internal basic ability to achieve moisture. The "Anti-sagging power" means the skin's internal basic ability to support the skin from the inside. The "Barrier power" is related to moisture evaporation from the skin and means the skin's inner basic ability to provide a barrier against ultraviolet rays. The "Redness" means the skin's inner basic ability to suppress inflammation. The "Skin firmness retention power" is related to skin elasticity (rate of return) and means the skin's internal basic ability to produce firmness. The "Softness" is related to skin elasticity (amplitude maximum) and means the skin's inner basic ability to achieve flexibility.

In this embodiment, the results of the amino acid component analysis are accumulated in advance using sweat from a considerable number of living organisms (people), linking the results with data on water content, transpiration rate, hemoglobin concentration, elasticity, etc. measured with other measurement devices. Then, using the accumulated data, a correlation equation between the amino acid composition ratio and the skin condition of the subject is obtained. In doing so, we do not simply seek a correlation between the data, but rather, based on the knowledge of dermatology and dermatology medicine, we use the components (free amino acids) of the tissue fluid (extracellular fluid) to infer the state inside the cells. This enables highly accurate estimation of each indicator even with a small amount of accumulated data of living organisms (about 100 persons).

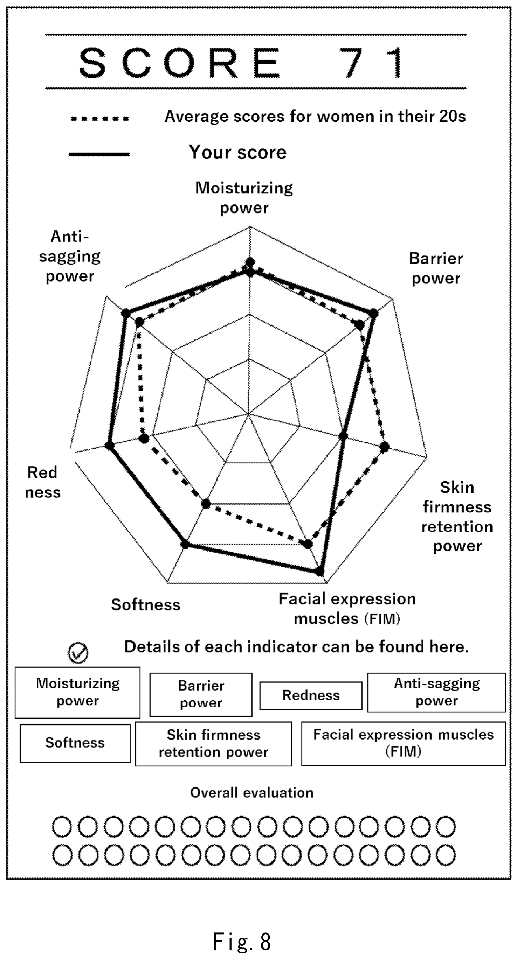

Figure 8 shows an example of information provided to users via a smartphone app regarding seven indicators: "Moisturizing power", "Anti-sagging power", "Barrier power", "Redness", "Skin firmness retention power", "Softness", "Facial expression muscles (FIM)". As shown in Figure 8, this example presents the seven indicators in the form of a radar chart. It also allows comparison with the average measurement results for women in their 20s. By providing information in the format shown in Figure 8, users can more intuitively understand the condition of the skin of subject. Additionally, when collecting sweat, by moving specific muscles while attaching the patch 3 near those muscles, it is also possible to obtain information about the condition of those muscles.

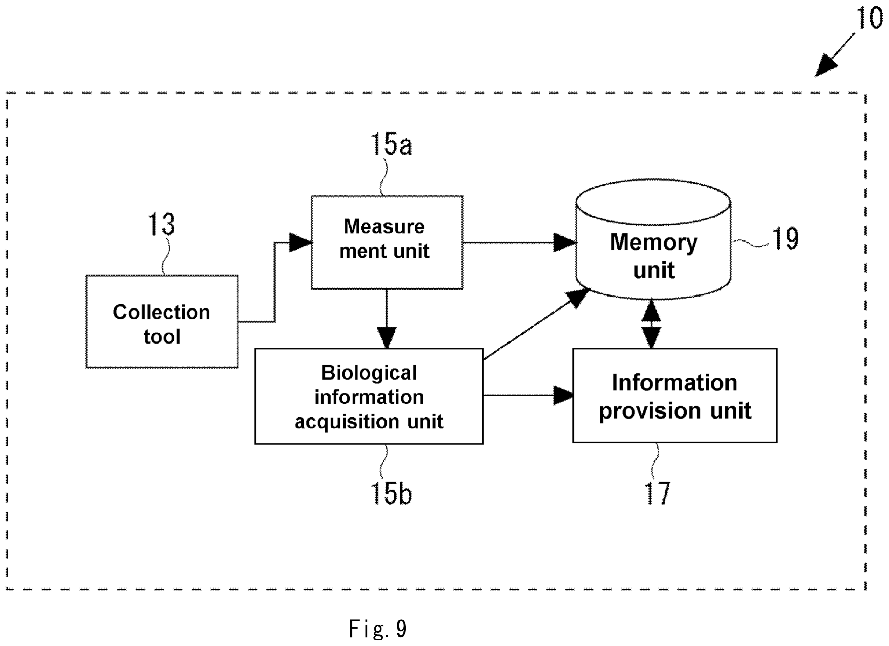

Figure 9 shows a functional block diagram of the present embodiment when viewed as a examination system for living organisms. In the present embodiment, the examination system 10 includes the collection tool 13 for collecting sweat samples and the measurement unit 15a for measuring the amino acid profile of the amino acids contained in the sweat collected by the collection tool 13. Additionally, the examination system 10 includes the biological information acquisition unit 15b that acquires biological information such as the types and degrees of deficiency of amino acids and hidden diseases based on the amino acid profile measured by the measurement unit 15a, and an information provision unit 17 that provides the biological information. Furthermore, the examination system 10 includes a memory unit 19 that stores measurement data from the measurement unit 15a, biological information obtained by the biological information acquisition unit 15b, and data on the content provided to users by the information provision unit 17.

The collection tool 13 in Figure 9 corresponds to a device for collecting sweat from the subject 1, such as the patch 3. The measurement unit 15a corresponds to the liquid chromatograph device 12. The biological information acquisition unit 15b corresponds to a server (not shown) equipped with a computing device for performing amino acid profile analysis and analysis using structural equation modelling. The information provision unit 17 corresponds specifically to the communication unit in a server (not shown) or similar device. The memory unit 19 corresponds specifically to a non-volatile memory device such as an external memory device or an external HDD owned by a server (not shown) or similar device.

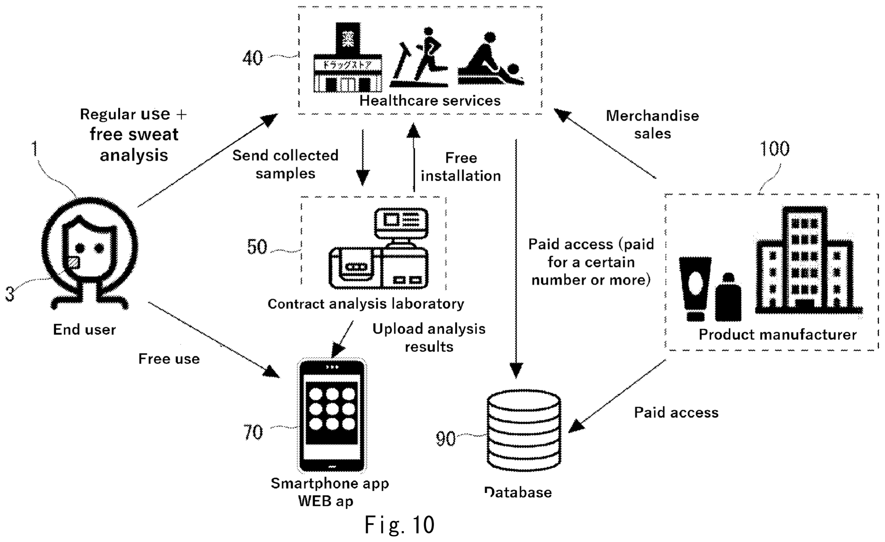

Next, using Figure 10, we will explain an example of the actual operational form of the examination method or examination system in this embodiment. In this operational form, a contracted analysis laboratory 50 in the figure acts as the business entity and requests the collection of sweat samples from healthcare service stores 40, such as drugstores, training gyms, and massage parlours. In this case, the healthcare service store 40 introduces the sweat analysis service free of charge from the contracted analysis laboratory 50. The end user, who is the subject 1, uses the healthcare service store 40 such as a training gym as usual and applies for the free sweat analysis. Then, at the healthcare service store 40, the end user receives the patch 3 as the collection tool 13, attaches the patch 3 to the examination site and collects sweat.

Next, the healthcare service store 40 that collected the end user's sweat sends the collected sample to the contracted analysis laboratory 50. The method of sending may be by mail, courier service, or direct collection by the contracted analysis laboratory 50, among others. At the contracted analysis laboratory 50, amino acid profiling measurements are performed using the liquid chromatograph device 12 as the measurement unit 15a. Additionally, using servers or PCs within the lab as the biological information acquisition unit 15b, analysis is performed using structural equation modelling. The contracted analysis lab 50 uploads the analysis results to the server, making the analysis results viewable by the end user via a separately prepared smartphone app or web app. The end user then launches the smartphone app or web app on a device such as a smartphone 70 and views the results on a display screen as described in Figure 7A and Figure 7B.

Furthermore, the contracted analysis laboratory 50 stores the analysis result data linked to the subject 1 in the memory unit 19 and constructs a database 90. The healthcare service stores 40 can access this database 90 for a fee. Additionally, supplement product manufacturers 100 can sell products to the healthcare service stores 40 and can also access the database 90 for a fee as information for product development within their own companies. Here, the process of storing the analysis result data linked to the subject 1 in the memory unit 19 corresponds to the memory step. The process of providing access to the database 90 corresponds to the set information provision step, and the communication system for providing access to the database 90 corresponds to the set information provision unit.

In this operational model, the operating entity, the contracted analysis laboratory 50, can generate revenue by providing information (big data) from the database 90, which was created through the free sweat analysis service, to the healthcare service stores 40 and the product manufacturers 100 for a fee. In addition, as the number of users of smartphone apps and web apps increases, it is also possible to generate advertising revenue. On the other hand, the healthcare service stores 40 can benefit from increased membership and improved brand recognition through the implementation of the free sweat analysis service. Furthermore, the product manufacturers 100 can benefit from expanded demand for supplements and other products based on this operational model.

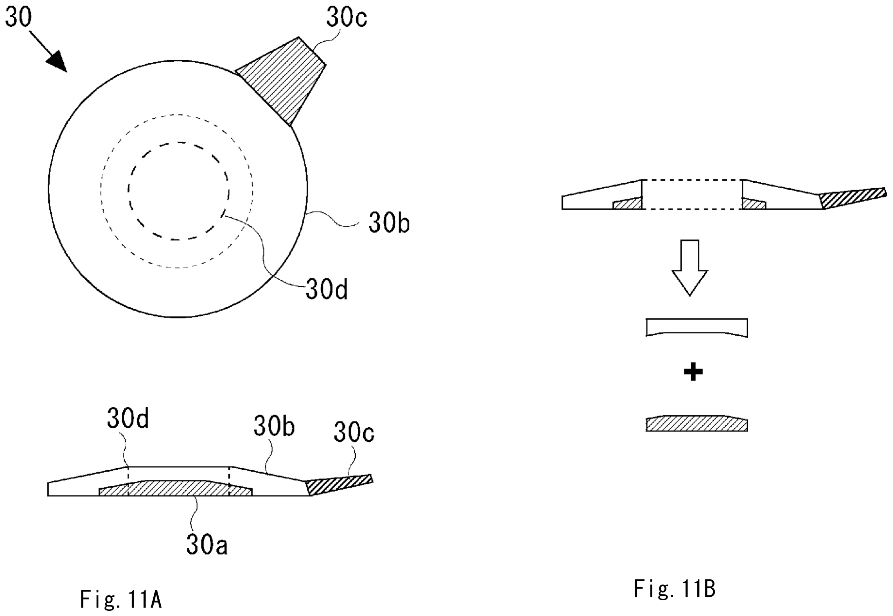

Next, Figure 11A and Figure 11B show schematic diagrams of the sweat-absorbing patch 30, which is the specific structure of the patch 3 in this embodiment. The upper part of Figure 11A is a top view of the sweat-absorbing patch 30, and the lower part is a cross-sectional view from the side. As shown in Figure 11A, the sweat-absorbing patch 30 has a roughly circular plate shape when viewed from above. It has a structure where the moisture-absorbing sheet 30a, which is an example of moisture-absorbing portion made of a moisture-absorbing material such as filter paper or moisture-absorbing polymer, is covered from above by an adhesive sheet 30b. Here, the lower surface of the adhesive sheet 30b in the figure is the adhesive surface. Therefore, the adhesive properties of the adhesive surface allow the moisture-absorbing sheet 30a and the adhesive sheet 30b to be bonded together, enabling the sweat-absorbing patch 30 to be attached to the skin of the subject 1. The thickness of the moisture-absorbing sheet 30a may be, for example, several hundred micrometres.

Additionally, the handle portion 30c protruding outward is provided on the outer periphery of the adhesive sheet 30b. After attaching the sweat-absorbing patch 30 to the skin of the subject 1 and collecting sweat, the collector can peel it off the skin by grasping the handle portion 30c with their fingers. The presence of the handle portion 30c reduces the risk of the collector touching the moisture-absorbing sheet 30a with their fingers. This prevents the moisture-absorbing sheet 30a from absorbing finger sweat, thereby suppressing a decrease in analysis accuracy. The handle portion 30c corresponds to the holding portion in this embodiment. This is also the case in the specific structure shown in the following figure.

In addition, the perforations 30d are provided on the moisture-absorbing sheet 30a and the adhesive sheet 30b as a separation mechanism. Furthermore, no adhesive surface is provided on the inner side of the perforations 30d on the adhesive sheet 30b. Therefore, as shown in Figure 11B, during analysis in the liquid chromatograph device 12, the analyst can separate a part surrounded by the perforation 30d, where the adhesive surface is not provided on the adhesive sheet 30b, from the sweat-absorbing patch 30 and obtain the part of the adhesive sheet 30b and the part of the moisture-absorbing sheet 30a separately. As a result, the analyst can obtain only the moisture-absorbing sheet 30a and make it easier to prepare samples for the liquid chromatograph device 12.

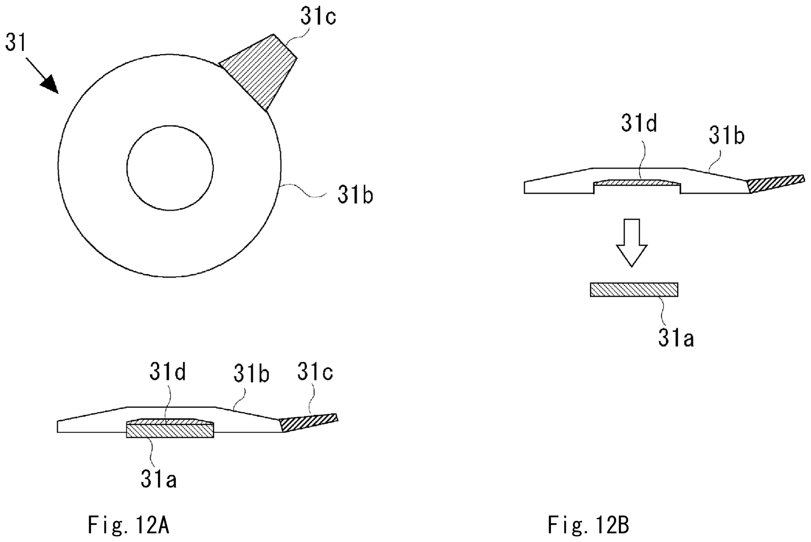

Figure 12A and Figure 12B show a schematic diagram of the sweat-absorbing patch 31 as a second specific structure of the patch 3. The upper part of Figure 12A is a top view of the sweat-absorbing patch 31, and the lower part is a cross-sectional view from the side. As shown in Figure 12A, the sweat-absorbing patch 31 has the same structure as the sweat-absorbing patch 30, in that it is covered with an adhesive sheet 31b on top of the moisture-absorbing sheet 31a and has the handle portion 31c. The difference between the sweat-absorbing patch 31 and the sweat-absorbing patch 30 lies in the separation mechanism. In the sweat-absorbing patch 31, the perforation 30d is not formed. Instead, the moisture-absorbing sheet 31a has a double-layer structure. The moisture-absorbing sheet 31a is held in place by overlapping with the second moisture-absorbing sheet 31d. The moisture-absorbing sheet 31a and the second moisture-absorbing sheet 31d are weakly bonded. Therefore, as shown in Figure 12B, during analysis in the liquid chromatograph device 12, the analyst can prepare the sample for the liquid chromatograph device 12 by peeling off the moisture-absorbing sheet 31a from the second moisture-absorbing sheet 31d. In this case, as shown in Figure 12B, the second moisture-absorbing sheet 31d remains on the adhesive sheet 31b side.

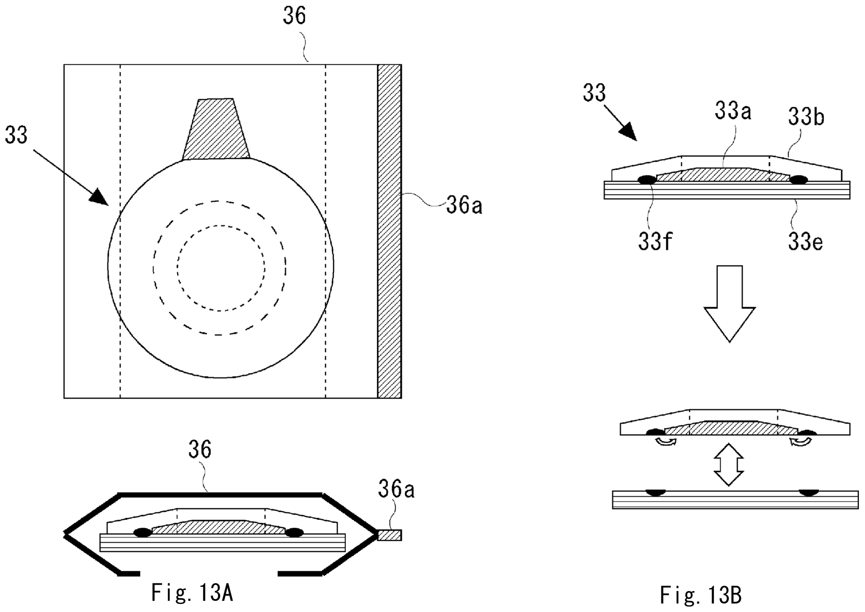

Figure 13A and Figure 13B show the third specific structure of the patch 3, namely the sweat-absorbing patch 33. The upper part of Figure 13A is a top view of the sweat-absorbing patch 33, and the lower part is a cross-sectional view from the side. Figure 13B is a diagram showing the usage situation. As shown in Figure 13B, the structure where the sweat-absorbing patch 33 is covered by the adhesive sheet 33b on top of the moisture-absorbing sheet 33a is the same as that of the sweat-absorbing patch 30. The difference between the sweat-absorbing patch 33 and the sweat-absorbing patch 30 lies in the fact that the sweat-absorbing patch 33 is stored in the storage case 36. The storage case 36 is equipped with the sealing zipper 36a that has a sealing function. By storing the sweat-absorbing patch 33 in the storage case 36 after sweat collection and closing the sealing zipper 36a, the sealing properties of the storage case 36 can be maintained, thereby preventing the sweat-absorbing patch 33 from drying out or becoming contaminated with foreign matters. Additionally, the storage case 36 may be structured to be sealed regardless of whether the sealing zipper 36a is opened or closed before sweat collection. In this case, the storage case 36 may first be opened with scissors or the like during sweat collection and subsequently sealed using the sealing zipper 36a.

In the example shown in Figure 13A and Figure 13B, the moisture-absorbing sheet 33a and the adhesive sheet 33b are stored in the storage case 36 in a state where they are attached to the base sheet 33e. The base sheet 33e prevents the adhesive strength of the adhesive sheet 33b from decreasing before use. Note that, although omitted in Figures 11 and 12, the sweat-absorbing patches 31 and 32 are also maintained in a state where they are attached to the base sheet before and after sweat collection.

In the example shown in Figure 13A and Figure 13B, the bag-shaped liquid container 33f containing the collection liquid is further provided between the adhesive sheet 33b and the base sheet 33e. When peeling the moisture-absorbing sheet 33a and adhesive sheet 33b from the base sheet 33e for sweat collection, the liquid container 33f ruptures, causing the collection liquid to leak out and be absorbed by the moisture-absorbing sheet 33a. Here, when collecting sweat, the collection efficiency of amino acids in sweat is higher when the moisture-absorbing sheet 33a is somewhat moist. In this example, it is possible to automatically moisten the moisture-absorbing sheet during the action of peeling off the moisture-absorbing sheet 33a and the adhesive sheet 33b from the base sheet 33e, thereby more easily improving the efficiency of collecting amino acids from sweat. The liquid container 33f in Figure 13B corresponds to the moisture supply container. Note that in the example of Figure 13A and Figure 13B, the liquid container 33f is positioned between the adhesive sheet 33b and the base sheet 33e, but it may also be positioned between the moisture-absorbing sheet 33a and the base sheet 33e, or between the moisture-absorbing sheet 33a and the adhesive sheet 33b.

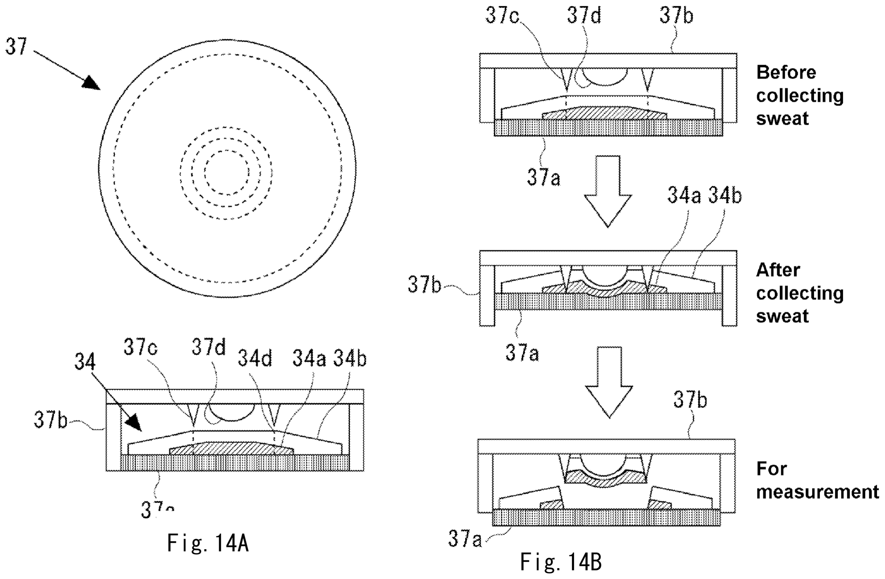

Next, Figure 14A and Figure 14B shows the sweat-absorbing patch 34 as the fourth specific structure of the patch 3. As shown in Figure 14A, in this example, the sweat-absorbing patch 34 consists of the moisture-absorbing sheet 34a and an adhesive sheet 34b. The sweat-absorbing patch 34 has the circular perforation 34d when viewed from above as a separation mechanism. In the example of Figure 14A and Figure 14B, the sweat-absorbing patch 34 is stored in the storage case 37. This storage case 37 has an overall flat cylindrical shape and consists of the base portion 37a in the shape of a circular plate and the lid portion 37b with a circular plate-shaped ceiling at the upper end of the cylindrical side. The sweat-absorbing patch 34 is attached to the base portion 37a of the storage case 37 using the adhesive force of the adhesive sheet 34b. Additionally, the lid portion 37b of the storage case 37 is equipped with a sharp protrusion called the separation protrusion 37c. This separation protrusion 37c is formed in a circular shape at a position opposite the perforation 34d when viewed from above. Additionally, the dome-shaped protrusion 37d is provided on the inner side of the separation protrusion 37c.

In this example, the upper part of Figure 14B shows the state before sweat collection. In this state, the base portion 37a and the lid portion 37b are relatively separated and weakly bonded. During sweat collection, the moisture-absorbing sheet 34a and the adhesive sheet 34b of the sweat-absorbing patch 34 are peeled off from the base portion 37a and attached to the examination site. Next, the middle part of Figure 14B shows the state after sweat collection. In the middle part of Figure 14B, the sweat-absorbing patch 34, after sweat collection, is reattached to the base portion 37a. The base portion 37a is pressed into the lid portion 37b, resulting in a relatively close distance between the two. As a result, the separation protrusion 37c deeply penetrates the perforation 34d of the sweat-absorbing patch 34 and separates the perforation 34d. This separation protrusion 37c corresponds to the separation assist mechanism.

At the same time, the protrusion 37d sandwiches the separated the moisture-absorbing sheet 34a and adhesive sheet 34b between the base portion 37a and itself in the vertical direction. The side surface of the separation protrusion 37c is in contact with the cut surface of the perforation 34d. This more reliably prevents the moisture-absorbing sheet 34a after sweat collection from drying out or becoming contaminated with foreign matters. It also suppresses the formation of bubbles in the moisture-absorbing sheet 34a. This protrusion 37d corresponds to the sealing portion.

During analysis in the liquid chromatograph device 12, as shown in the lower part of Figure 14B, by separating the lid portion 37b and the base portion 37a, it is possible to separate the part of the sweat-absorbing patch 34 necessary for sample preparation while keeping it on the lid portion 37b side. This prevents the analyst from coming into contact with the moisture-absorbing sheet 34a at this stage, enabling the sample for the liquid chromatograph device 12 to be prepared more easily and reliably.

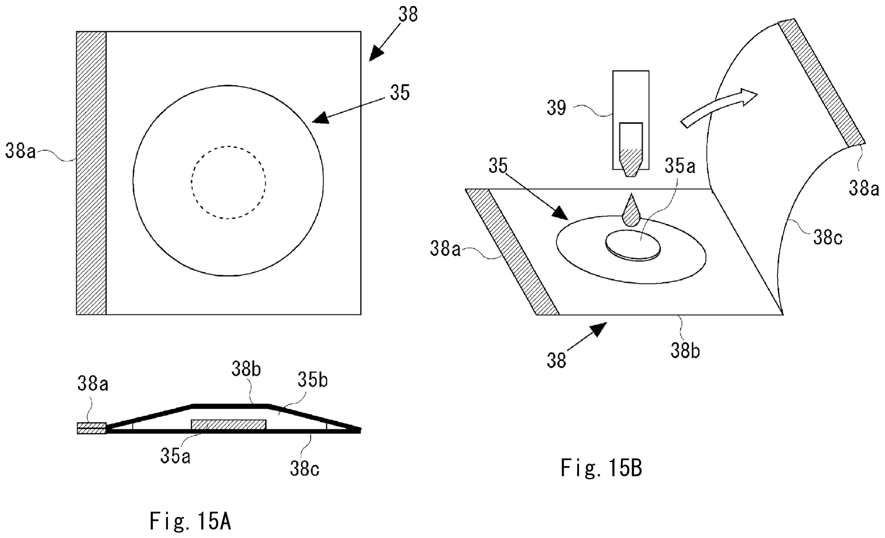

Figure 15A and Figure 15B show the fifth specific structure of the patch 3, namely the sweat-absorbing patch 35. The upper part of Figure 15A is a top view of the sweat-absorbing patch 35 stored in a packaging seal 38, and the lower part is a cross-sectional view from the side. Figure 15B is a diagram showing the condition of the sweat-absorbing patch 35 when it is ready for use. As shown in Figure 15A, in the initial state, the sweat-absorbing patch 35 is stored in the packaging seal 38. The packaging seal 38 wraps the sweat-absorbing patch 35 by sandwiching it between the sealing cover sheet 38b and the sealing base sheet 38c. Additionally, the surface of the moisture-absorbing sheet 35a of the sweat-absorbing patch 35 is fixed to the sealing base sheet 38c by the adhesive force of the adhesive sheet 35b. Furthermore, the surface opposite the moisture-absorbing sheet 35a of the sweat-absorbing patch 35 is fixed to the sealing cover sheet 38b by the adhesive force of the sealing cover sheet 38b.

Furthermore, the adhesive force between the surface of the moisture-absorbing sheet 35a of the sweat-absorbing patch 35 and the sealing base sheet 38c is weaker than the adhesive force between the surface opposite the moisture-absorbing sheet 35a of the sweat-absorbing patch 35 and the sealing cover sheet 38b. As shown in Figure 15B, by holding and pulling the respective ends 38a of the sealing cover sheet 38b and the sealing base sheet 38c, it is possible to peel off the moisture-absorbing sheet 35a side of the sweat-absorbing patch 35 from the sealing base sheet 38c and expose the moisture-absorbing sheet 35a.

In this example, the purified water-filled syringe 39 provided to the user along with the sweat-absorbing patch 35 is used to moisten the moisture-absorbing sheet 35a. In this case, the purified water-filled syringe 39 provided to the user is pre-filled with the necessary amount of purified water, and by dripping all the purified water in the purified water-filled syringe 39 onto the moisture-absorbing sheet 35a, the moisture-absorbing sheet 35a can be appropriately moistened.

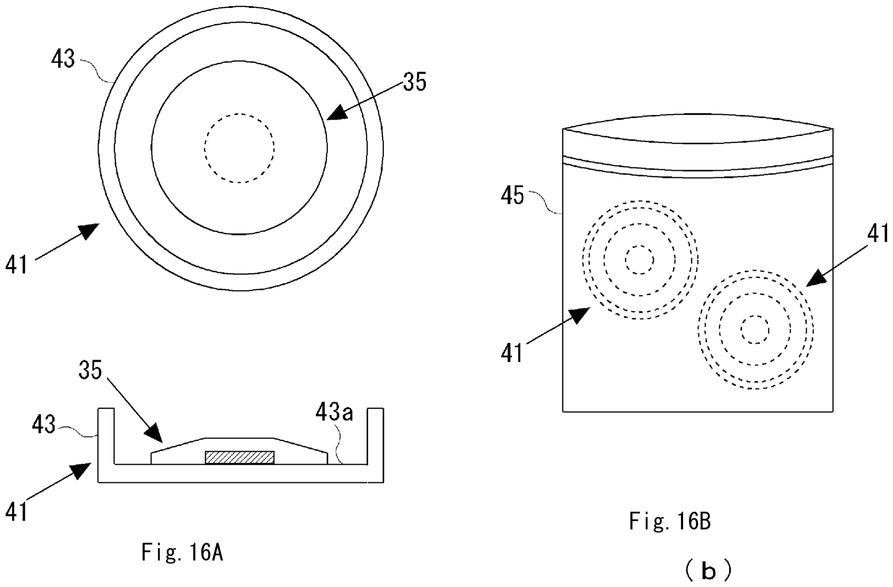

Next, Figure 16A and Figure 16B show the state of the sweat-absorbing patch 35 after sweat collection when it is retrieved for measuring the amino acid profile. Figure 16A is a diagram showing the state of the sweat-absorbing patch 35 stored in the storage case 43 after sweat collection (hereinafter referred to as the "patch-stored case 41"). The upper part of Figure 16A shows a top view, and the lower part shows a cross-sectional view from the side. In this example, the storage case 43 has a flat, bottomed cylindrical shape with one side closed. The bottomed cylindrical shape ensures sufficient strength and eliminates the need for operations such as opening and closing the lid, thereby improving operability. In this example, the storage case 43 is made of resin, but it is not limited to resin. Materials with high strength and low moisture absorption, such as glass, can also be used. Furthermore, the cross-section of the storage case 43 viewed from the axial direction is not limited to a circular shape. The sweat-absorbing patch 35 is fixed to the inner side of the bottom surface 43a of the storage case 43 by adhering the moisture-absorbing sheet 35a side to the surface and securing it with the adhesive force of the adhesive sheet 35b. In this state, transporting and storing the sweat-absorbing patch 35 after sweat collection allows the components of sweat sample to be stably maintained for at least one month or more.

Figure 16B shows the state of the sweat-absorbing patch 35 during transportation. As shown in Figure 16B, during transportation, multiple patch-stored cases 41 are placed inside the return bag 45. After accommodating all patch-stored cases 41 to be transported, the return bag 45 is sealed and transported. In this process, while some parts of the storage case 43 may come into contact with the sweat-absorbing patches 35 stored in other storage cases 43, contact between the sweat-absorbing patches 35 is prevented, and the simple structure of the storage case 43 prevents collected sweat from mixing.

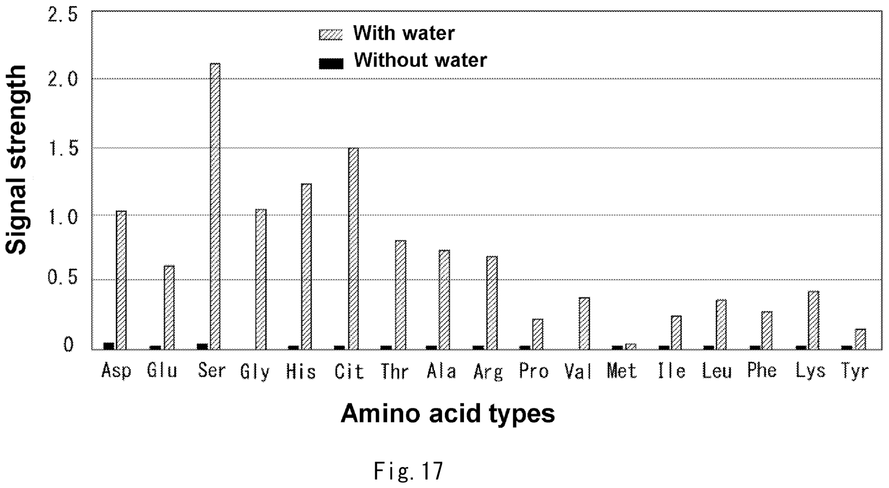

Note that in the above description, an example was explained where the moisture-absorbing sheet 35a is moistened with purified water before attaching the sweat-absorbing patch 35 to the examination site to collect the sweat of the subject. This is based on the findings from the inventors' research that pre-moistening the moisture-absorbing sheet 35a with water improves the collection efficiency for nearly all amino acids. Figure 17 shows a graph with the horizontal axis representing the type of amino acid and the vertical axis representing the signal strength obtained by the liquid chromatograph device 12. In the bar graphs for each amino acid, black indicates the case without water, and hatching indicates the case with water. As shown in Figure 17, for nearly all types of amino acids, the case with water (where the moisture-absorbing sheet 35a was moistened with water before attachment) yields significantly higher signal strength compared to the case without water.

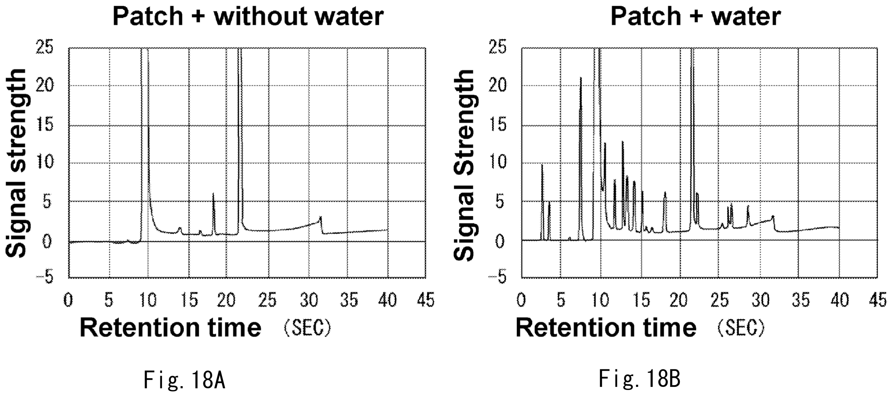

Figure 18A and Figure 18B show examples of amino acid profiles obtained in the case with and without water. Figure 18A shows the amino acid profile in the case without water, and Figure 18B shows the amino acid profile in the case with water. Comparing the two profiles, it can be understood that in the case with water shown in Figure 18B, there are more detectable peaks, and more precise measurements are possible compared to the case without water. Note that the units of signal strength in Figures 17 and 18 are different from those in Figure 5, and the signal strength values in Figures 5, 17, and 18 cannot be directly compared across figures.

In the above embodiment, we explained an example of obtaining information about the condition of the skin, muscles, allergies, mental disorders, diseases, sleep, and inflammation by analysing the amino acid profile as a biological component using the liquid chromatograph device 12. For example, amino acids closely related to the condition of the skin include serine, glycine, and alanine. Amino acids closely related to muscle condition include branched-chain amino acids such as valine, leucine, and isoleucine. Amino acids closely related to sleep information include glycine and tryptophan. Amino acids closely related to recovery information include free amino acids such as ornithine. Amino acids closely related to mental condition include tyrosine.

In addition, examples of the biological components analysed in this disclosure include proteins related to bodily condition, vitamins as essential components related to vitality, taurine related to vitality, and hyaluronic acid related to beauty and moisture, in addition to amino acids. Furthermore, examples include amyloid beta related to dementia, examples include human growth hormone related to metabolism and muscle, examples include L-carnitine related to vitality and metabolism, examples include coenzyme Q10 related to vitality and aging, and examples include minoxidil and keratin related to vasodilation and hair growth. Furthermore, examples include collagen, collagen peptides, elastin, and elastin peptides related to muscle and skin tissue, and examples include glucosamine and chondroitin related to cartilage.

In addition, as a method for measuring the biological component profile in the measurement unit 15a, methods related to liquid chromatography (LC), such as high-performance liquid chromatography (HPLC) and liquid chromatography/mass spectrometry (LC/MS), may also be used. Furthermore, as a method for detecting biological components in the detection section 120d of the liquid chromatograph device 12, methods such as fluorescence detection, ultraviolet absorption spectroscopy, or nuclear magnetic resonance spectroscopy may be used, or alternatively, methods such as mass spectrometry or QCM (quartz crystal microbalance) may be employed using the external detector 125.

### [Explanation of symbols]

- 1: Subject
- 3: Patch
- 5: Analysis device
- 7: Display screen
- 10: Examination system
- 12: Liquid chromatograph
- 13: Collection tool
- 15a: Measurement unit
- 15b: Biological information acquisition unit
- 17: Information provision unit
- 19: Memory unit
- 30, 31, 33, 34, 35: Sweat-absorbing patch
- 37, 43: Storage case

## Claims

1. A method of examining a living organism and providing biological information on an examination subject by analyzing biological components in sweat of the examination subject which is the living organism, the method comprising:
a collection step of collecting the sweat at an examination site in the examination subject;
a measurement step of measuring biological components profile of the sweat collected in the collection step;
a biological information acquisition step of acquiring, based on the biological components profile measured in the measurement step, the biological information on the examination subject; and
an information provision step of providing the biological information on the examination subject, acquired in the biological information acquisition step, in a predetermined format.

2. The method of examining a living organism according to claim 1, wherein the biological information on the examination subject includes information on a state of a skin or a muscle of the examination subject at the examination site, the information being acquired based on the biological components profile.

3. The method of examining a living organism according to claim 1, wherein the biological information on the examination subject includes information on a state of any one of disease, sleep, and inflammation of the examination subject, the information being acquired based on the biological components profile.

4. The method of examining a living organism according to claim 1, wherein the biological information on the examination subject includes biological information on the examination subject, which is acquired from an amount of the biological components constituting the biological components profile.

5. The method of examining a living organism according to claim 1, wherein the biological information on the examination subject includes biological information on the examination subject, which is acquired by analyzing, based on a structural equation modelling method, the biological components profile.

6. The method of examining a living organism according to claim 1, wherein the biological information on the examination subject includes biological information on the examination subject, which is acquired by analyzing the biological components profile by machine learning.

7. The method of examining a living organism according to claim 1, wherein
the biological information on the examination subject includes an amount of an individual biological component constituting the biological components profile, and
the information provision step provides information on at least one of a type of an insufficient or excessive biological component in the examination subject and a method of resolving the insufficiency or excess.

8. The method of examining a living organism according to claim 1, further comprising:
a memory step of storing the biological information on the examination subject in association with the examination subject; and
a set information provision step of providing, as a set, the biological information for a set of a plurality of the examination subjects.

9. The method of examining a living organism according to claim 1, wherein, in the collection step, the sweat is collected from the examination subject by attaching a patch to the examination site, wherein the patch includes
a moisture-absorbing portion capable of absorbing liquid, and
an adhesive sheet having a larger area than an area of the moisture-absorbing portion, the adhesive sheet having an adhesive surface on at least one surface thereof, the adhesive surface covering the moisture-absorbing portion such that the moisture-absorbing portion is held to be attachable to the examination site.

10. The method of examining a living organism according to claim 9, wherein the moisture-absorbing portion is moistened with water before the patch is attached to the examination site.

11. The method of examining a living organism according to claim 9, wherein, in the collection step, an attachment location of the patch is any one of a cheek, a temple, a forehead, a back side of a mandibular angle, and a rear side of a neck of the examination subject, or a combination thereof.

12. The method of examining a living organism according to claim 9, wherein the patch after the sweat is collected in the collection step is moved in a state in which the patch is attached to an inside of a bottom surface of a flat bottomed cylindrical case with one side closed, and the measurement step is performed.

13. The method of examining a living organism according to any one of claims 1 to 12, wherein the biological components contain at least one of amino acids, proteins, bases, lipids, and extracellular vesicles.

14. A patch adapted to collect, in the collection step of the method of examining a living organism according to claim 1, the sweat at the examination site in the examination subject, the patch comprising:
a moisture-absorbing portion capable of absorbing liquid; and
an adhesive sheet having a larger area than an area of the moisture-absorbing portion, the adhesive sheet having an adhesive surface on at least one surface thereof, the adhesive surface covering the moisture-absorbing portion such that the moisture-absorbing portion is held to be attachable to the examination site.

15. The patch according to claim 14, further comprising a separation mechanism configured to facilitate, when a part of the moisture-absorbing portion is cut off to prepare a sample in the measurement step, separation of the part of the moisture-absorbing portion.

16. The patch according to claim 14, further comprising a holding portion capable of holding the adhesive sheet when the adhesive sheet is peeled off from the examination site.

17. The patch according to claim 14, further comprising:
a base sheet adapted to protect the moisture-absorbing portion by allowing the adhesive surface of the adhesive sheet to be attached to the base sheet before and after use; and
a bag-shaped moisture supply container adapted to contain collection liquid therein, the moisture supply container being disposed between the moisture-absorbing portion and the adhesive sheet or between the moisture-absorbing portion or the adhesive sheet and the base sheet,
wherein, when the adhesive sheet and the moisture-absorbing portion are peeled off from the base sheet, the moisture supply container is broken to enable moisture supply to the moisture-absorbing portion.

18. A storage container adapted to store therein the patch according to claim 14, the storage container comprising
an sealing portion adapted to, when the storage container is deformed, suppress drying of the moisture-absorbing portion by allowing an inner surface of the sealing portion to contact the moisture-absorbing portion or the adhesive sheet.

19. A storage container adapted to store therein the patch according to claim 14, the storage container comprising
a separation assist mechanism configured to, when the storage container is deformed, assist separation of a part of the moisture-absorbing portion by the separation mechanism.

20. An examination system for a living organism, configured to provide biological information on an examination subject by analyzing biological components in sweat of the examination subject which is the living organism, the examination system comprising:
a collection tool configured to collect the sweat at an examination site in the examination subject;
a measurement unit configured to measure a biological components profile of the sweat collected by the collection tool;
a biological information acquisition unit configured to acquire, based on the biological components profile measured by the measurement unit, the biological information on the examination subject; and
an information provision unit configured to provide the biological information on the examination subject, acquired by the biological information acquisition unit, in a predetermined format.

21. The examination system for a living organism according to claim 20, wherein the biological information on the examination subject includes information on a state of a skin or a muscle of the examination subject, the information being acquired based on the biological components profile.

22. The examination system for a living organism according to claim 20, wherein the biological information on the examination subject includes information on any one of disease, sleep, and inflammation of the examination subject, the information being acquired based on the biological components profile.

23. The examination system for a living organism according to claim 20, wherein the biological information on the examination subject includes biological information on the examination subject, which is acquired from an amount of the biological components constituting the biological components profile.

24. The examination system for a living organism according to claim 20, wherein the biological information on the examination subject includes biological information on the examination subject, which is acquired by analyzing, based on a structural equation modelling method, the biological components profile.

25. The examination system for a living organism according to claim 20, wherein the biological information on the examination subject includes biological information on the examination subject, which is acquired by analyzing the biological components profile by machine learning.

26. The examination system for a living organism according to claim 20, wherein
the biological information on the examination subject includes an amount of an individual biological component constituting the biological components profile, and
the information provision unit further provides information on at least one of a type of an insufficient or excessive biological component in the examination subject and a method of resolving the insufficiency or excess.

27. The examination system for a living organism according to claim 20, further comprising:
a storage unit configured to store therein the biological information on the examination subject in association with the examination subject; and
a set information provision unit configured to provide, as a set, the biological information for a set of the plurality of examination subjects.

28. The examination system for a living organism according to claim 20, wherein
the collection tool has a moisture-absorbing portion capable of absorbing liquid, and
the sweat is collected by attaching the collection tool to the examination subject such that the moisture-absorbing portion that has absorbed collection liquid in advance comes into contact with the examination site.

29. The examination system for a living organism according to any one of claims 20 to 28, wherein the biological components contain at least one of amino acids, proteins, bases, lipids, and extracellular vesicles.
